# DEMANDE DE BREVET EUROPEEN

(11) **EP 1 247 492 A1**
(43) Date de publication de la demande: **09.10.2002**
(21) Numéro de dépôt: 01810346.5
(22) Date de dépôt: 06.04.2001
(51) Int. Cl.: A61B 17/04, A61B 17/06

(54) **Instrument endoscopique de suture**

(71) Demandeur: Biomedix S.A., 1701 Fribourg (CH)
(72) Inventeur: Godin, Norman, 1208 Geneve (CH)
(74) Mandataire: Savoye, Jean-Paul

(57) **Abrégé**

Cet instrument endoscopique de suture, comprend un élément tubulaire (1) dont une extrémité distale comporte une aiguille de suture (5) et dont l'extrémité proximale comporte un organe d'actionnement (3) pour commander ladite aiguille de suture (5), des moyens de transmission (2) reliant ledit organe d'actionnement (3) à ladite extrémité distale à travers ledit élément tubulaire (1) flexible qui comporte, à proximité de ladite extrémité distale, un élément gonflable (8) et du côté de ladite extrémité proximale, une source de fluide de gonflage (9). Un conduit (10) relie ledit élément gonflable (8) à ladite source de fluide de gonflage (9). Cette source de fluide de gonflage est agencée pour permettre de maintenir sélectivement ledit élément gonflable (8) à un volume et une pression déterminés.

## Description

La présente invention se rapporte à un instrument endoscopique de suture, comprenant un élément tubulaire dont une extrémité distale comporte une aiguille de suture et dont l'extrémité proximale comporte un organe d'actionnement pour commander ladite aiguille de suture, des moyens de transmission reliant ledit organe d'actionnement à ladite extrémité distale à travers ledit élément tubulaire.

Un tel instrument est décrit dans différents brevets, notamment dans le EP-0 674 875. Il permet de réaliser des sutures sur des tissus situés à l'intérieur d'un organisme vivant en disposant l'extrémité distale de l'instrument contre le tissu à suturer. En sortant latéralement du corps allongé de l'instrument, l'aiguille courbe de suture peut traverser le tissu de l'organe dans lequel la suture doit être réalisée. Ensuite on peut ressortir le fil de suture du corps creux de l'instrument pour faire un noeud que l'on pousse avec l'extrémité de l'instrument puis que l'on serre. Il s'agit là d'une technique de nouage connue dans les interventions en chirurgie laparoscopique.

Le corps allongé de cet instrument est rigide, de sorte qu'il n'est pas adapté à être utilisé en passant le long d'un conduit naturel de l'organisme étant donné qu'un tel conduit n'est généralement pas rectiligne. Tel est le cas en particulier de l'accès à l'oesophage au travers de la bouche notamment, de sorte que cet appareil n'est pas adapté à la pose d'une prothèse pour s'opposer au reflux gastrique, telle que celle décrite dans le WO 96/29954.

Avec un instrument dont le corps allongé est flexible, le problème vient du fait qu'il n'est plus possible d'exercer une force apte à faire pénétrer l'aiguille de suture à travers la paroi de la prothèse et celle de l'oesophage, comme avec un instrument dont le corps est rigide.

Le but de la présente invention est d'apporter une solution à ce problème.

A cet effet, la présente invention a pour objet un instrument endoscopique de suture selon la revendication 1.

L'avantage de cet instrument est d'être flexible lui permettant de s'adapter à la morphologie du conduit dans lequel il est introduit, tout en permettant, une fois en place, de l'appuyer contre la paroi de ce conduit avec une force suffisante pour empêcher le recul de l'extrémité distale de l'instrument lorsque l'aiguille de suture perce les parois du conduit et de la prothèse destinée à être fixée à l'intérieur de ce conduit.

Le dessin annexé illustre, très schématiquement et à titre d'exemple, une forme d'exécution de l'instrument endoscopique de suture objet de la présente invention, illustrant, dans cet exemple, la fixation d'une prothèse destinée à empêcher le reflux gastrique.
La figure 1 est une vue d'ensemble de cet instrument;
La figure 2 est une vue en coupe selon la ligne II-II de la figure 1;
la figure 3 est une vue partielle agrandie de la partie distale de l'instrument illustré par la figure 1 introduite dans l'oesophage d'un patient représentant une première phase du processus de suture;
la figure 4 est une vue semblable à la figure 3, montrant une autre phase du processus de suture ;
la figure 5 est une vue semblable à la figure 3, montrant une dernière phase du processus de suture.

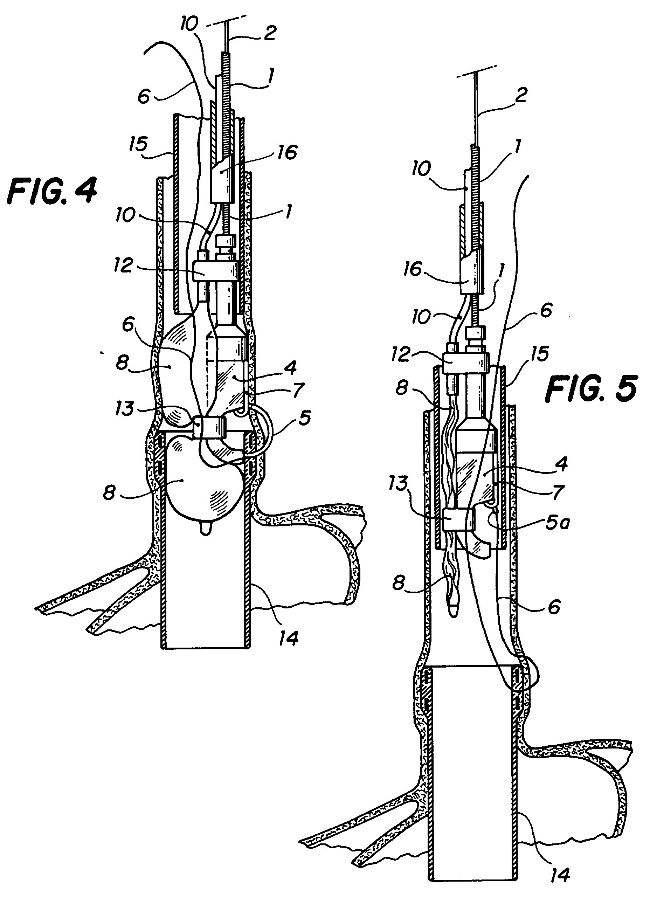

L'instrument endoscopique de suture comporte un élément tubulaire flexible 1 constitué par une gaine, notamment une gaine formée par un ressort à boudin à l'intérieur de laquelle coulisse un élément de transmission 2 qui peut être constitué par un fil métallique ou plastique, voire par un câble. A une extrémité proximale de la gaine tubulaire 1, l'élément de transmission 2 est solidaire d'un organe d'actionnement 3, tandis que l'extrémité distale de la gaine tubulaire 1 est solidaire d'un organe de guidage 4 d'une aiguille de suture 5 de forme semi-circulaire. Une extrémité de cette aiguille de suture 5 est solidaire de l'extrémité distale de l'élément de transmission 2, tandis que son autre extrémité comporte une pointe amovible 5a de forme conique (figure 4) à laquelle est fixé une extrémité du fil de suture 6. Une grille de retenue 7 de la pointe amovible 5a est solidaire de l'organe de guidage 4 et est destinée à permettre à la pointe amovible 5a de forme conique de pénétrer à travers la grille de retenue 7, mais de ne pas pouvoir ressortir, de sorte que lorsque l'aiguille 5 est retirée en arrière en exerçant une traction sur l'organe d'actionnement 3, elle se détache de la pointe 5a qui reste prisonnière de la grille de retenue 7 avec le fil de suture 6.

L'extrémité distale de l'instrument endoscopique de suture comporte un élément gonflable 8 formé par un ballonnet relié à une petite pompe à main 9 par un conduit souple 10. Une valve 11 permet de faire communiquer le conduit 10 et donc le ballonnet 8 avec l'atmosphère pour le dégonfler jusqu'à ce qu'il atteigne un volume et une pression déterminés.

Selon une variante, la pompe à main 9 et la valve 11 peuvent avantageusement être remplacées par une seringue (non représentée), raccordée à l'extrémité proximale du conduit souple 10, permettant d'envoyer de l'air ou un liquide dans le ballonnet 8, puis de retirer cet air ou ce liquide en actionnant alternativement le piston de la seringue en avant puis en arrière effectuant ainsi un simple transfert du fluide de gonflage, de sorte que la valve 11 n'est plus nécessaire. En outre, avec du liquide comme fluide de gonflage, celui-ci étant incompressible, il n'y a qu'à le transférer dans le ballonnet 8 lors du gonflage et le retirer dans la seringue pour dégonfler ce ballonnet 8, tout ceci sans pression supérieure à la pression atmosphérique.

Deux organes de fixation 12, 13 servent à fixer et à positionner le ballonnet 8 et le conduit 10 sur l'organe de guidage 4 de l'aiguille de suture 5. Le ballonnet 8 est fixé à l'organe de guidage 4 de manière à occuper une position située à l'opposé de la trajectoire décrite par l'aiguille de suture 5, pour appliquer la partie de cet organe de guidage 4 d'où sort l'aiguille de suture 5 contre la paroi d'une prothèse 14 pour empêcher le reflux gastrique, destinée à être cousue à la base de l'oesophage comme illustré. La forme de ce ballonnet 8 est choisie de préférence pour qu'à l'état gonflé, sa section transversale présente une forme de grain de haricot, de manière à épouser la forme de l'espace entre l'organe de guidage 4 et la paroi de l'oesophage.

En général, cette prothèse 14 sera fixée dans une hernie hiatale qui se forme entre la base de l'oesophage et l'estomac étant donné qu'une telle hernie est généralement associée aux problèmes de reflux gastrique que la prothèse 14 est destinée à contenir pour qu'il n'atteigne pas la paroi de l'oesophage, qui ne supporte pas l'acidité des liquides gastriques.

Grâce à la présence du ballonnet 8, l'organe de guidage 4 est appliqué contre la paroi de la prothèse 14 avec une force suffisante pour permettre à l'aiguille de suture 5 de traverser la paroi de la prothèse 14 ainsi que celle de l'oesophage ou de la hernie hiatale à laquelle la prothèse 14 doit être fixée.

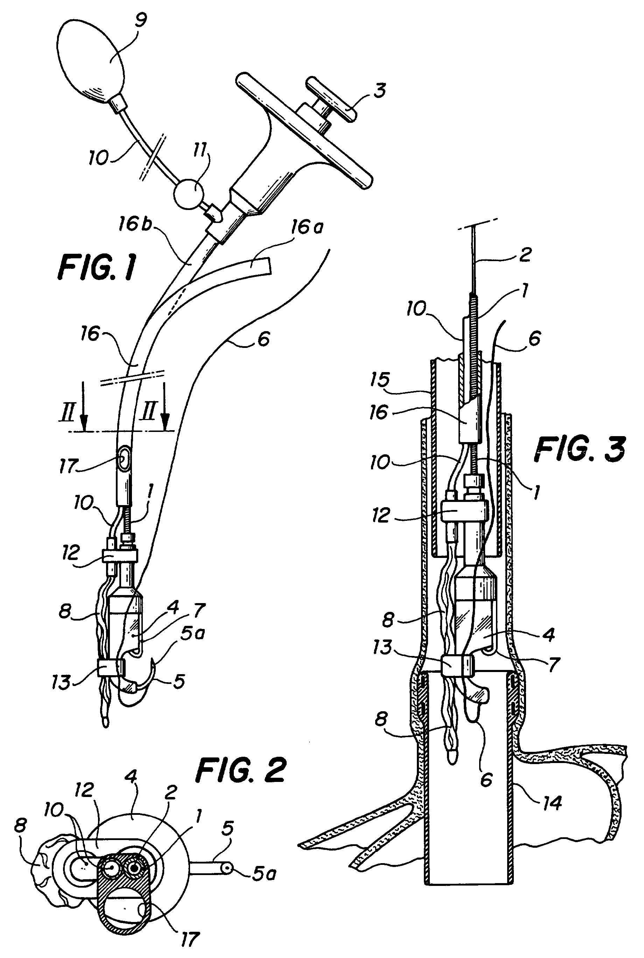

Avantageusement, comme illustré plus particulièrement par les figures 1 et 2, l'élément tubulaire flexible 1 et le conduit 10 sont montés dans deux canaux ménagés dans un corps souple 16, dans lequel passe un troisième canal 17 destiné à un endoscope (non représenté). Comme illustré par la figure 1, le corps souple 16 se divise en allant vers l'extrémité proximale en deux parties, l'une 16a comprenant le canal 17 de l'endoscope, l'autre 16b comprenant les deux canaux pour le passage de la gaine souple 1 et du conduit 10.

De préférence, l'instrument de suture endoscopique monté dans le corps souple 16 est amené à la base de l'oesophage à travers un tube de guidage flexible 15. Le positionnement de l'organe de guidage 4 peut se faire soit à l'aide de l'endoscope (non représenté) amené à travers le canal 17 du corps souple 16, soit par radioscopie.

Lorsque le fil de suture 6 est ressorti à l'intérieur de l'oesophage, comme illustré par la figure 5 et que la pointe amovible 5a à laquelle il est fixé est retenue par la grille 7, le ballonnet 8 est dégonflé à l'aide de la valve 11 ou en retirant le piston de la seringue en arrière et l'instrument peut alors être ressorti à travers le tube de guidage 15 pour faire un noeud avec le fil de suture 6, puis pour le descendre et le serrer. Cette opération peut être répétée en fonction du nombre de noeuds que l'on désire effectuer pour fixer la prothèse 14.

## Revendications

1. Instrument endoscopique de suture, comprenant un élément tubulaire (1) dont une extrémité distale comporte une aiguille de suture (5) et dont l'extrémité proximale comporte un organe d'actionnement (3) pour commander ladite aiguille de suture (5), des moyens de transmission (2) reliant ledit organe d'actionnement (3) à ladite extrémité distale à travers ledit élément tubulaire (1), **caractérisé en ce que** cet élément tubulaire (1) est flexible et qu'il comporte, à proximité de ladite extrémité distale, un élément gonflable (8) et du côté de ladite extrémité proximale, une source de fluide de gonflage (9), un conduit (10) reliant ledit élément gonflable (8) à ladite source de fluide de gonflage (9), qui est agencée pour permettre de maintenir sélectivement ledit élément gonflable (8) à un volume et une pression déterminés.

2. Instrument selon la revendication 1, dans lequel ladite extrémité distale dudit élément tubulaire (1) comporte des moyens de guidage (4) d'une aiguille de suture courbe (5) pour guider cette dernière selon une trajectoire centrée sensiblement au centre de sa courbure et s'étendant latéralement hors dudit élément tubulaire (1) et dans lequel ledit élément gonflable (8) occupe une position latérale par rapport audit élément tubulaire (1) et sensiblement opposée à ladite trajectoire de ladite aiguille de suture (5).

3. Instrument selon l'une des revendications précédentes, dans lequel la section droite dudit élément gonflable (8) à l'état gonflé présente sensiblement la forme d'un grain de haricot.

4. Instrument selon l'une des revendications précédentes, dans lequel ledit élément tubulaire flexible (1) et ledit conduit (10) reliant ledit élément gonflable (8) à ladite source de fluide de gonflage (9) sont disposés à l'intérieur de canaux ménagés dans un corps souple (16), lequel comporte encore un canal (17) pour le passage d'un endoscope.

5. Instrument selon l'une des revendications précédentes, dans lequel ladite source de fluide de gonflage est constituée par une seringue et ledit fluide est du liquide.
